(19) Europäisches Patentamt
European Patent Office
Office européen des brevets

(11) **EP 4 369 350 A1**

(12) **EUROPEAN PATENT APPLICATION**

(43) Date of publication:
**15.05.2024 Bulletin 2024/20**

(21) Application number: **23209641.2**

(22) Date of filing: **14.11.2023**

(51) International Patent Classification (IPC):
***G16H 20/17*** (2018.01)    ***G16H 40/63*** (2018.01)

(52) Cooperative Patent Classification (CPC):
**G16H 20/17; G16H 40/63**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **14.11.2022 US 202263383595 P**

(71) Applicant: **Insulet Corporation**
**Acton MA 01720 (US)**

(72) Inventors:
• **LEE, Joon Bok**
  **Acton, MA (US)**
• **ZHENG, Yibin**
  **Hartland, WI (US)**
• **O'CONNOR, Jason**
  **Acton, MA (US)**
• **ZADE, Ashutosh**
  **San Diego, CA (US)**

(74) Representative: **Peterreins Schley**
**Patent- und Rechtsanwälte PartG mbB**
**Hermann-Sack-Straße 3**
**80331 München (DE)**

(54) **MAXIMUM SUBCUTANEOUS INSULIN ABSORPTION RATES TO CALCULATE EFFECTIVE INSULIN-ON-BOARD IN AUTOMATED INSULIN DELIVERY SYSTEMS**

(57)    A drug delivery system including a memory storing programming code operable to enable delivery of insulin and a processor operable to execute the programming code. When executed, the programming code causes the processor to: calculate a first aggregate insulin-on-board amount for a user based on a plurality of individual insulin-on-board estimates made over a first period of time, determine that the first aggregate insulin-on-board amount is greater than a maximum depot formation threshold amount, determine a first residual amount of insulin remaining in the first aggregate insulin-on-board amount, increase a subsequent calculation of an individual insulin-on-board estimate based on the first residual amount of insulin, and utilize the subsequent calculation of the individual insulin-on-board estimate in a calculation of a second aggregate insulin-on-board amount over a second period of time.

**FIG. 1**

EP 4 369 350 A1

**Description**

**CROSS-REFERENCE TO RELATED APPLICATIONS**

This application claims priority to and the benefit of U.S. Provisional App. No. 63/383,595, filed November 14, 2022, the entirety of which is incorporated by reference herein.

**TECHNICAL FIELD**

The disclosed embodiments generally relate to medication delivery. More particularly, the disclosed embodiments relate to techniques and processes that incorporate maximum subcutaneous insulin absorption rates to calculate effective insulin-on-board in automated insulin delivery systems and devices.

**BACKGROUND**

Subcutaneous insulin deliveries for people with diabetes (e.g., Type 1 or Type 2 Diabetes) often lead to over-delivery of insulin due to significant delays between insulin delivery and insulin action (or utilization). The concept of insulin-on-board (IOB) can be utilized to mitigate the risk of such over-deliveries, but IOB relies on an approximated heuristic that assumes insulin is "utilized" by the body at an ever increasing rate with increased insulin injections. This approximated heuristic does not consider the possibility of a saturated rate of absorption. In some cases, additional insulin delivery beyond saturation may lead to an insulin depot formation that can take a significant period of time to absorb or fully dissolve, leading to an increased risk of over-delivery.

Accordingly, there is a need for a more accurate technique to determine the rate of insulin delivery from automated insulin delivery systems and devices.

**SUMMARY**

At least one aspect of the present disclosure is directed to a drug delivery system. The drug delivery system includes a memory storing programming code operable to enable delivery of insulin and a processor operable to execute the programming code. When executed, the programming code causes the processor to: calculate a first aggregate insulin-on-board amount for a user based on a plurality of individual insulin-on-board estimates made over a first period of time, determine that the first aggregate insulin-on-board amount is greater than a maximum depot formation threshold amount, determine a first residual amount of insulin remaining in the first aggregate insulin-on-board amount, increase a subsequent calculation of an individual insulin-on-board estimate based on the first residual amount of insulin, and utilize the subsequent calculation of the individual insulin-on-board estimate in a calculation of a second aggregate insulin-on-board amount over a second period of time.

A non-transitory computer readable medium embodied with programming code that causes a processor to: calculate a first aggregate insulin-on-board amount for a user based on a plurality of individual insulin-on-board estimates made over a first period of time, determine that the first aggregate insulin-on-board amount is greater than a maximum depot formation threshold amount, determine a first residual amount of insulin remaining in the first aggregate insulin-on-board amount, increase a subsequent calculation of an individual insulin-on-board estimate based on the first residual amount of insulin, and utilize the subsequent calculation of the individual insulin-on-board estimate in a calculation of a second aggregate insulin-on-board amount over a second period of time.

Another aspect of the present disclosure is directed to a drug delivery system. The drug delivery system includes a memory storing programming code operable to enable delivery of insulin and a processor operable to execute the programming code. When executed, the programming code causes the processor to: calculate an effective insulin-on-board value based on a maximum decay rate, determine a residual amount of insulin using the calculated effective insulin-on-board value and an insulin-on-board estimate for a current operational cycle, produce an aggregate insulin-on-board amount for a subsequent operational cycle using utilizing the residual amount of insulin and a history of insulin deliveries, and set a constraint on a calculation of an optimum insulin delivery rate based on the aggregate insulin-on-board amount.

**BRIEF DESCRIPTION OF THE DRAWINGS**

In the drawings, like reference characters generally refer to the same parts throughout the different views. In the following description, various embodiments of the present disclosure are described with reference to the following drawings, in which:

**FIG. 1** illustrates a block diagram of a drug delivery system according to embodiments of the present disclosure;

**FIG. 2A** illustrates a block diagram of a drug delivery system according to embodiments of the present disclosure;

**FIG. 2B** illustrates a perspective view of an example of a drug delivery system of FIG. 2A according to embodiments of the present disclosure;

**FIG. 3** illustrates a flow diagram of an example for determining an aggregate insulin-on-board (IOB) amount for a user;

**FIG. 4** illustrates a plot of example IOB amounts for a user over time;

**FIG. 5** illustrates a flow diagram of an example for determining a residual amount of insulin remaining in an aggregate IOB amount;

**FIG. 6** illustrates a flow diagram of an example for calculating an optimum insulin delivery rate and delivering insulin at the optimum insulin delivery rate;

**FIG. 7** illustrates a flow diagram of an example for calculating an effective insulin-on-board value; and

**FIG. 8** illustrates an example computing device.

The drawings are not necessarily to scale. The drawings are merely representations, not intended to portray specific parameters of the disclosure. The drawings are intended to depict exemplary embodiments of the disclosure, and therefore are not to be considered as limiting in scope. Furthermore, certain elements in some of the figures may be omitted, or illustrated not-to-scale, for illustrative clarity. Still furthermore, for clarity, some reference numbers may be omitted in certain drawings.

## DETAILED DESCRIPTION

Systems, devices, and methods in accordance with the present disclosure will now be described more fully hereinafter with reference to the accompanying drawings, where one or more embodiments are shown. The systems, devices, and methods may be embodied in many different forms and are not to be construed as being limited to the embodiments set forth herein. Instead, these embodiments are provided so the disclosure is thorough and complete, and fully conveys the scope of methods and devices to those skilled in the art. Each of the systems, devices, and methods disclosed herein provides one or more advantages over conventional systems, components, and methods.

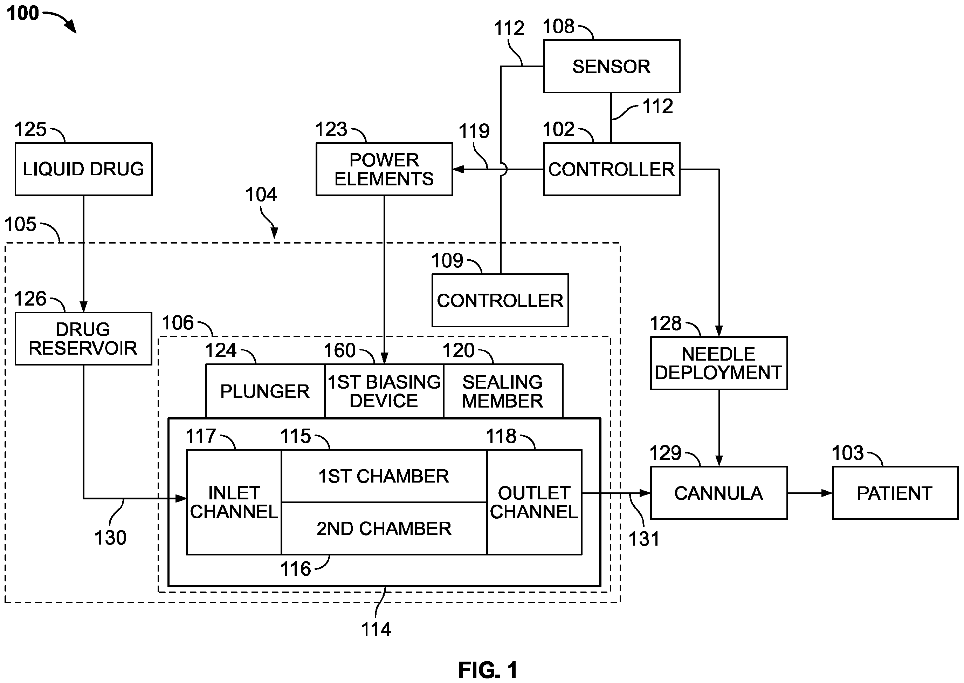

FIG. 1 illustrates a simplified block diagram of an example system 100. The system 100 may be a wearable or on-body drug delivery device and/or an analyte sensor attached to the skin of a patient 103. The system 100 may include a controller 102, a pump mechanism 104 (hereinafter "pump 104"), and a sensor 108. The sensor 108 may be one or more of a glucose and/or other analyte sensor such as, for example, a continuous glucose monitor, a ketone sensor, a heart rate monitor, a blood oxygen sensor element, or the like, and may be incorporated into the wearable device. The sensor(s) 108 may, for example, be operable to measure blood glucose (BG) values of a user to generate a measured BG level signal 112. The controller 102, the pump 104, and the sensor(s) 108 may be communicatively coupled to one another via a wired or wireless communication path. For example, each of the controller 102, the pump 104 and the sensor(s) 108 may be equipped with a wireless radio frequency transceiver operable to communicate via one or more communication protocols, such as Bluetooth®, or the like. As described in greater detail herein, the system 100 may also include a pump 104 which includes a drive mechanism 106 having at least one housing 114 defining a pump chamber 115, an inlet channel 117, an outlet channel 118, and optionally a second chamber 116. The drive mechanism 106 may further include a resilient sealing member 120 enclosing the pump chamber 115, and a first biasing device 160 (e.g., shape memory alloy (SMA) wire or the like) operable to bias or move a plunger 124 relative to the pump chamber 115, as described in greater detail herein. In an example, the drive mechanism 106, the controller 109 and the drug reservoir 126 may be incorporated in at least one housing 105, which may be one or more parts that may be a combination of reusable and disposable parts. The at least one housing 105 may be formed from one or more materials, such as a plastic, a metal, or the like. The system 100 may include additional components not shown or described for the sake of brevity.

The controller 102 may receive a desired BG level signal, which may be a first signal, indicating a desired or target BG level or range for the patient 103. The desired BG level or range may be stored in memory of a controller 109 on pump 104, received from a user interface of the controller 102, or another device, or by an algorithm within controller 109 (or controller 102). Alternatively, a target BG level or range may be pre-programmed into memory of controller 109 without requiring user input. The sensor(s) 108 may be coupled to the patient 103 and operable to measure an approximate value of a BG level of the user. In response to the measured BG level or value, the sensor(s) 108 may generate a signal indicating the measured BG value. As shown in the example, the controller 102 may also receive from the sensor(s) 108 via a communication path, the measured BG level signal 112, which may be a second signal.

Based on the desired BG level and the measured BG level signal 112, the controller 102 or controller 109 may generate one or more control signals for directing operation of the pump 104. For example, one control signal 119 from the controller 102 or controller 109 may cause the pump 104 to turn on, or activate one or more power elements 123 operably connected with the pump 104. In the case where the first biasing device 160 is an SMA wire, activation of the SMA wire by the power element 123 may cause the SMA wire to change shape and/or length, which in turn may cause movement of the plunger 124 and the resilient sealing member 120 via a gearing mechanism. The specified amount of a liquid drug 125 (e.g., insulin, GLP-1, pramlintide, or a co-formulation of insulin, GLP-1 or pramlintide; glucagon; a chemotherapy drug; a blood thinner; a pain medication; an arthritis drug; or the like) may be drawn from the reservoir 126 into the pump chamber 115, through the inlet channel 117, in response to a change in pressure due to the change in configuration of the resilient sealing member 120 and the plunger 124. In some examples, the specified amount of the liquid drug 125 to be delivered may be determined based on a difference between the desired BG level and the actual BG level signal 112. For example, specified amount of the liquid drug 125 may be determined as an appropriate amount of insulin to drive the measured BG level of the user toward the desired BG level. Based on operation of the pump 104, as determined by the control signal 119, the patient 103 may receive the liquid drug from a reservoir 126. The system 100 may operate as a closed-loop system, an open-loop system, or as a hybrid system. In an exemplary closed-loop system, the controller 109 may direct operation of the pump 104 without input from the user or controller 102, and may receive BG level signal 112 from the sensor(s) 108. The sensor(s) 108 may be housed within the pump 104 or may be housed in a separate device and communicate wirelessly directly with the pump 104 (e.g., with controller 109) and/or with an external controller 102.

As further shown, the system 100 may include a needle deployment component 128 in communication with the controller 102 or the controller 109. Though shown separately, needle deployment component 128 may be integrated within pump 104. The needle deployment component 128 may include a needle and/or cannula 129 deployable into the patient 103 and may have one or more lumens and one or more holes at a distal end thereof. The cannula 129 may form a portion of a fluid path coupling the patient 103 to the reservoir 126. More specifically, the inlet channel 117 may be coupled to the reservoir 126 by a first fluid path component 130. The first fluid path component 130 may be of any size and shape and may be made from any material. The first fluid path component 130 enables fluid, such as the liquid drug 125 in the reservoir 126, to be transferred to the drive mechanism 106.

As further shown, the outlet channel 118 may be coupled to the cannula 129 by a second fluid path component 131. The second fluid path component 131 may be of any size and shape and may be made from any material. The second fluid path component 131 may be connected to the cannula 129 to allow fluid expelled from the pump 104 to be provided to the patient 103. The first and second fluid path components 130 and 131 may be rigid, flexible, or a combination thereof.

The controller 102/109 may be implemented in hardware, software, or any combination thereof. The controller 102/109 may, for example, be a processor, a logic circuit or a microcontroller coupled to a memory. The controller 102/109 may be a processor that includes a memory. The controller 102/109 may maintain a date and time as well as provide other functions (e.g., calculations or the like) performed by processors. The controller 102/109 may be operable to execute an artificial pancreas (AP) algorithm stored in memory (not shown in this example) that enables the controller 102/109 to direct operation of the pump 104. For example, the controller 102/109 may be operable to receive an input from the sensor(s) 108, wherein the input comprises analyte level data, such as blood glucose data or levels over time. Based on the analyte level data, the controller 102/109 may modify the behavior of the pump 104 and resulting amount of the liquid drug 125 to be delivered to the patient 103.

The power elements 123 may be a battery, a supercapacitor, a piezoelectric device, a combination thereof, or the like, for supplying electrical power to the pump 104. In other embodiments, the power element 123, or an additional power source (not shown), may also supply power to other components of the pump 104, such as the controller 102, memory, the sensor(s) 108, and/or the needle deployment component 128.

In an example, the sensor(s) 108 may be a device communicatively coupled to the controller 102 and may be operable to measure a blood glucose value at a predetermined time interval, such as approximately every 5 minutes, 1 minute, or the like. The sensor(s) 108 may provide a number of blood glucose measurement values to the AP application.

In some embodiments, the pump 104, when operating in a normal mode of operation, provides insulin stored in the reservoir 126 to the patient 103 based on information (e.g., blood glucose measurement values, target blood glucose values, insulin on board, prior insulin deliveries, time of day, day of the week, inputs from an inertial measurement unit, global positioning system-enabled devices, Wi-Fi-enabled devices, or the like) provided by the sensor(s) 108 or other functional elements of the system 100 or pump 104. For example, the pump 104 may contain analog and/or digital circuitry that may be implemented at the controller 102/109 for controlling the delivery of the drug or therapeutic agent. The circuitry used to implement the controller 102/109 may include discrete, specialized logic and/or components, an application-specific integrated circuit, a microcontroller or processor that executes software instructions, firmware, programming instructions or programming code enabling, for example, an AP application stored in memory, or any combination thereof. For example, the controller 102/109 may execute a control algorithm and other programming code that may make the controller 102/109 operable to cause the pump to deliver doses of the drug or therapeutic agent to a user at predetermined intervals or as needed to bring blood glucose measurement values to a target blood glucose value. The size and/or timing of some of the doses may be pre-programmed, for example, into the AP application by the patient 103 or by a third party (such as a health care provider, a parent or guardian, a manufacturer of the wearable drug delivery device, or the like) using a wired or wireless link, or may be calculated iteratively by the controller 102 or controller 109, such as every 5 minutes.

Although not shown, in some embodiments, the sensor(s) 108 may include a processor, memory, a sensing or measuring device, and a communication device. The memory of the sensor(s) 108 may store an instance of an AP application as well as other programming code and be operable to store data related to the AP application.

In various embodiments, the processor of the sensor(s) 108 may include discrete, specialized logic and/or components, an application-specific integrated circuit, a microcontroller or processor that executes software instructions, firmware, programming instructions stored in memory, or any combination thereof.

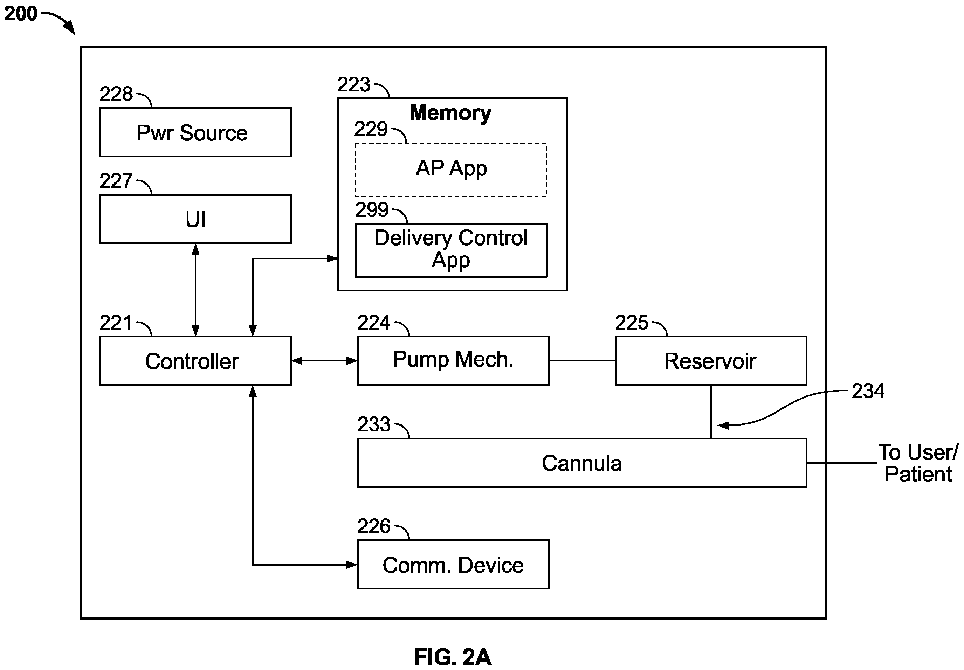

FIG. 2A illustrates a simplified block diagram of a drug delivery system 200. The drug delivery system 200 may include a controller 221, a memory 223, an AP application 229 and delivery control application 299 stored in the memory 223, a pump mechanism 224, a communication device 226, user interface 227, and a power source 228. The memory 223 may be operable to store programming code and applications including a delivery control application 299, the AP application 229, and data. The delivery control application 299 and the AP application 229 may optionally be stored on other devices.

The controller 221 may be coupled to the pump mechanism 224 and the memory 223. The controller 221 may include logic circuits, a clock, a counter or timer as well as other processing circuitry, and be operable to execute programming code and the applications stored in the memory 223 including the AP application 229 and/or the delivery control application 299. A communication device 226 may be communicatively coupled to the controller 221 and may be operable to wirelessly communicate with an external device, such as a personal diabetes management (PDM) device, a smart device such as a smartphone and/or a smartwatch, or the like.

For ease of discussion, the computer programs and computer applications that implement the medication delivery algorithms or applications may be referred to herein as an "AP application," a delivery control application," or an "automated insulin delivery (AID) algorithm."

The AP application 229 and/or the delivery control application 299 may be configured to provide automatic delivery of insulin based on an analyte sensor input, such as signals received from an analyte sensor, such as a continuous blood glucose monitor, or the like. The signals from the analyte sensor may contain blood glucose measurement values, timestamps, or the like. In addition, or alternatively, while examples may be described with reference to a closed loop algorithmic implementation, variations of the disclosed examples may be implemented to enable open loop use. The open loop implementations allow for use of different modalities of delivery of insulin such as smart pen, syringe, or the like in addition to the described drug delivery devices. For example, the disclosed AP application 229, the delivery control application 299, and related algorithms may be operable to perform various functions related to open loop operations, such as determination of a total daily setting for a drug or combination of drugs, such as a total daily insulin setting or the like.

The pump mechanism 224 may be operable to deliver a drug, like insulin, at a fixed or variable rate. For example, an AP application 229 or AID algorithm executing on a personal diabetes management (PDM) device or a smart phone may determine or be informed that a user's total daily insulin (e.g., bolus and/or basal deliveries) is 48 units per 24 hours, which may translate to an exemplary physiological dosage rate of 1 unit per hour that may be determined according to a diabetes treatment plan. However, the pump mechanism 224 may be operable to deliver insulin at rates different from the example physiological dosage rate of 1 unit per hour. In an example, the system 200 may be attached to the body of a user, such as a patient or diabetic via, for example, an adhesive, (e.g., directly attached to the skin of the user) and may deliver any therapeutic agent, including any drug or medicine, such as insulin, morphine, or the like, to the user. In an example, a surface of the system 200 may include an adhesive (not shown) to facilitate attachment to a user. Alternatively, the system 200 may, for example, be worn on a belt or in a pocket of the user and the liquid drug may be delivered to the user via tubing to an infusion site on the user.

In various examples, the drug delivery system 200 may be an automatic, wearable drug delivery device. For example, the drug delivery system 200 may include a reservoir 225 configured to hold a liquid drug (such as insulin), a needle and/or cannula 233 for delivering the drug into the body of the user (which may be done subcutaneously, intraperitoneally, or intravenously), and a pump mechanism 224, or other drive mechanism, for transferring the drug from the reservoir 225, through a needle or cannula 233, and into the user.

The pump mechanism 224 may be fluidly coupled to reservoir 225, and communicatively coupled to the medical device controller 221. The pump mechanism 224 may be coupled to the reservoir 225 and operable to output the liquid drug from the reservoir 225 via a fluid delivery path and out of the cannula 233. The pump mechanism 224 may have mechanical parameters and specifications, such as a pump resolution, that indicate mechanical capabilities of the pump mechanism. The pump mechanism 224 may also have electrical connections to control circuitry (not shown) that is operable to control operation of the pump mechanism 224. The pump resolution is the amount of insulin the pump mechanism 224 delivers in a pump mechanism pulse, and a pulse is an actuation of the pump mechanism for a preset time period. Actuation may be when power from the power source 228 is applied to the control circuitry coupled to the pump mechanism 224 and the pump mechanism 224 operates to pump an amount of insulin in a preset amount of time from the reservoir 225 via one or more pulses. Alternatively, the pump mechanism 224 may be substantially mechanical in structure and operation and utilize mechanical energy storage devices, such as springs or other biasing members to operate the pump mechanism 224.

The cannula 233 of FIG. 2A may be coupled to the reservoir 225 via a fluid delivery path 234. The cannula 233 may be operable to output the liquid drug to a user when the cannula 233 is inserted in the user.

The drug delivery system 200 may also include a power source 228, such as a battery, a supercapacitor, a piezoelectric device, a combination thereof, or the like, that is operable to supply electrical power to the pump mechanism 224 and/or other components (such as the controller 221, memory 223, and the communication device 226) of the drug delivery system 200.

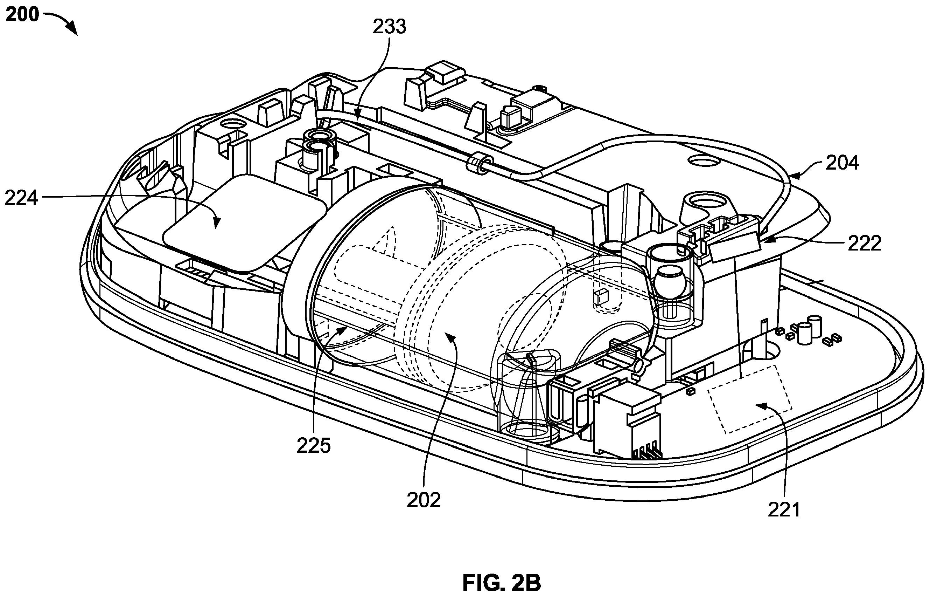

As shown in FIG. 2B, the drug delivery system 200 may include a plunger 202 positioned within the reservoir 225. An end portion or stem of the plunger 202 can extend outside of the reservoir 225. The pump mechanism 224 may, under control of the controller 221, be operable to cause the plunger 202 to expel the fluid, such as a liquid drug (not shown) from the reservoir 225 and into a fluid component 204 and cannula 233 by advancing into the reservoir 225. In various examples, a sensor, such as that shown at 222, may be integrated anywhere along the overall fluid delivery path of the drug delivery system 200, which includes the reservoir 225, the fluid delivery path component 204, and the cannula 233.

The controller 221 may be implemented in hardware, software, or any combination thereof. In various examples, the controller 221 can be implemented as dedicated hardware (e.g., as an application specific integrated circuit (ASIC)). The controller 221 may be a constituent part of the drug delivery system 200, can be implemented in software as a computational model, or can be implemented external to the drug delivery system 200 (e.g., remotely). The controller 221 may be configured to communicate with one or more sensors (e.g., sensor(s) 108 of FIG. 1 or sensor 222).

A reservoir, such as 225, may be included in a drug delivery device to store a liquid drug (e.g., insulin). For example, the reservoir 225 may be filled, or partially filled, with a liquid drug or a liquid drug solution. In one example, a liquid drug solution is a mixture of the liquid drug and added preservatives. The reservoir may store the liquid drug until all of the liquid drug has been dispensed (e.g., into a patient via a cannula). As such, the liquid drug (or solution) may remain in the reservoir for a period of time (e.g., 1 day, 3 days, 1 week, 2 weeks, etc.).

As discussed above, subcutaneous insulin deliveries for people with diabetes can lead to over-delivery of insulin due to delays between insulin delivery and insulin action (or utilization). The concept of insulin-on-board (IOB) is utilized to mitigate the risk of such over-deliveries. However, IOB relies on an approximated heuristic that assumes insulin is "utilized" by the body at an ever increasing rate with increased insulin injections. This approximated heuristic does not take into account the possibility of a saturated rate of absorption. Additional insulin delivery beyond saturation may lead to the formation of an insulin depot (e.g., an excess amount of insulin in the subcutaneous region near the cannula 233) that can take a significant period of time to fully absorb into the body. In some cases, the formation of an insulin depot can lead to an increased risk of over-delivery.

Automated insulin delivery (AID) devices may utilize IOB estimates to determine the delivery rate at which insulin is delivered to the user. In one example, the system 100 of FIG. 1 and the system 200 of FIG. 2 correspond to AID devices. In some examples, the delivery rate of the AID device is limited by a user-specific IOB constraint. For example, a maximum delivery amount may be calculated using Equation (1) below:

$$U_{max}(i) = IOB(i) - \frac{G(i) - target(i)}{CF(i)} \qquad \text{Equation (1)}$$

where, $i$ is the delivery cycle, $IOB(i)$ is the user's IOB at each respective delivery cycle, $G(i)$ is the user's glucose level at each respective delivery cycle, $target(i)$ is the target glucose concentration at each respective delivery cycle, $CF(i)$ is a correction factor, and $U_{max}(i)$ is the maximum delivery amount during delivery cycle ($i$). The user's glucose level $G(i)$ may be obtained using a continuous glucose monitor (CGM) (e.g., sensor 108 of FIG. 1). In some examples, the correction factor $CF(i)$ corresponds to 1800/TDI, where TDI is the user's total daily insulin dose.

In one example, the IOB of the user can be calculated using Equation (2) below:

$$IOB(i) = [I(i-1) \ \ I(i-2) \ ... \ ... \ I(i-h \cdot j - 1) \ \ I(i-h \cdot j)] \cdot D_h \qquad \text{Equation (2)}$$

where, $i$ is the delivery cycle, $I$ is the amount of insulin delivered at each respective cycle, $j$ is the number of cycles per hour (e.g., 12 or one cycle every 5 mins), $h$ is the number of hours over which insulin utilization is being forecasted (e.g., 2 hours, 2.5 hours, 4 hours, 4.7 hours, 6 hours, 8 hours, etc.), and $D_h$ is an hourly decay curve. In some embodiments, $h$ may be between about 2 hours to about 10 hours, about 3 hours to about 9 hours, and/or between about 4 hours to about 8 hours.

In some examples, the hourly decay curve $D_h$ is a linear decay curve that can be represented by Equation (3)

below:

$$D_h = \begin{bmatrix} 1 \\ 1 - \dfrac{1}{h \cdot j} \\ \vdots \\ 1 - \dfrac{h \cdot j - 1}{h \cdot j} \\ 0 \end{bmatrix} \qquad \text{Equation (3)}$$

where $h$ and $j$ represent the same factor as in Equation 2.
Accordingly, the written out equation (2) would read:

$$IOB(i) = [I(i-1) \quad I(i-2) \quad \ldots \quad \ldots \quad I(i - h \cdot j - 1) \quad I(i - h \cdot j)] \cdot \begin{bmatrix} 1 \\ 1 - \dfrac{1}{h \cdot j} \\ \vdots \\ 1 - \dfrac{h \cdot j - 1}{h \cdot j} \\ 0 \end{bmatrix} \text{Equation (2a)}$$

The hourly decay curve $D_h$ proportionally increases the amount of insulin that is assumed to be absorbed or utilized by the user's body. However, this absorption rate does not proportionally scale with larger insulin deliveries (e.g., above 100 $\mu$L). For example, subcutaneous insulin deliveries may experience a limit to the total amount of insulin that is actually absorbed by the delivery route in the user's body. Following subcutaneous insulin delivery, an insulin depot may be formed in the adipose tissue of the user's body at the injection site. The insulin depot corresponds to a reserve of insulin that is not immediately absorbed into the body. The surface area of the insulin depot may increase as insulin deliveries are provided. Once the total volume of insulin delivered reaches a threshold (e.g., above 100 $\mu$L), a volume of the insulin depot may begin to increase in such a manner that the body does not absorb the additional insulin more quickly (i.e., saturation occurs). For example, the vertical height (or depth) of the insulin depot may begin to increase rather than the surface area. In some cases, this increase in vertical height (or depth) can cause the user's rate of insulin absorption to saturate, leading to an increased risk of over-delivery as insulin in the body increases, but is not being absorbed, leading to a potential erroneous interpretation of blood glucose values by a conventional AID system.

As such, improved techniques for determining the rate of insulin delivery from AID systems and devices are provided herein. In at least one embodiment, an aggregate IOB amount for a user is calculated based on a plurality of individual IOB estimates made over a period of time. In one example, the calculated aggregate IOB amount is compared to a maximum depot formation threshold amount. In response to a determination that the calculated aggregate IOB amount is greater than the maximum depot formation threshold amount, a residual amount of insulin remaining in the calculated aggregate IOB amount is determined and used to increase a subsequent calculation of an individual IOB estimate. The term "subsequent calculation of an individual IOB estimate" may refer to one or more individual IOB estimate value and may also be referred to as "subsequent individual IOB estimate" or "plurality of subsequent individual IOB estimates". In some examples, the subsequent calculation of the individual IOB estimate is utilized in a calculation of a further aggregate IOB amount over a subsequent period of time.

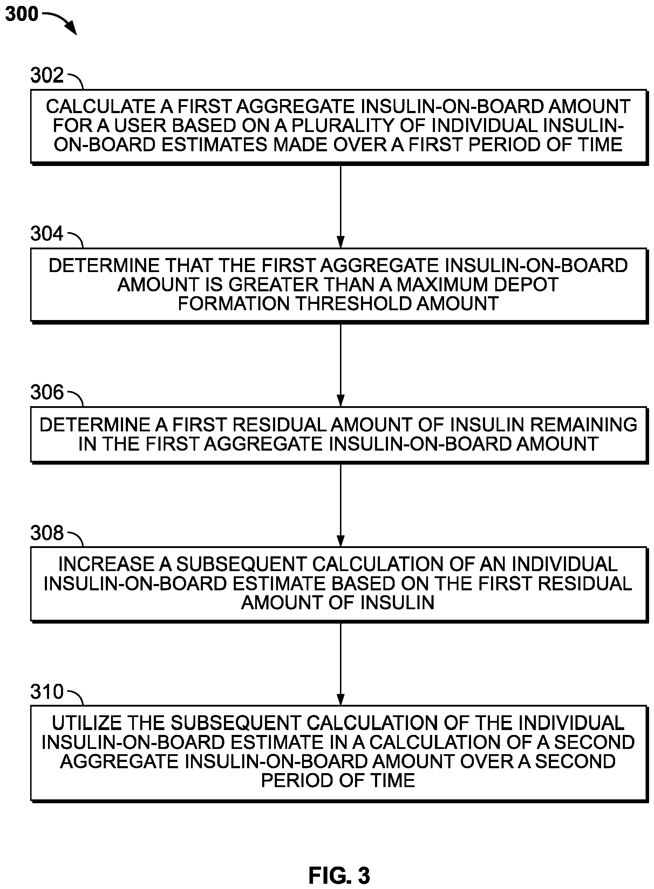

FIG. 3 is a flow diagram of an example method for determining an adjusted aggregate IOB amount for a user. In one example, the adjusted aggregate IOB amount is used to determine the rate of insulin delivery provided by an AID system. In some examples, the method 300 is performed, at least in part, by a controller or a processor of an AID system (e.g., controller 102 or controller 109 of FIG. 1 or controller 221 of FIG. 2). In other examples, the method 300 can be performed by controllers, processors, or computing systems that are external to the insulin delivery system.

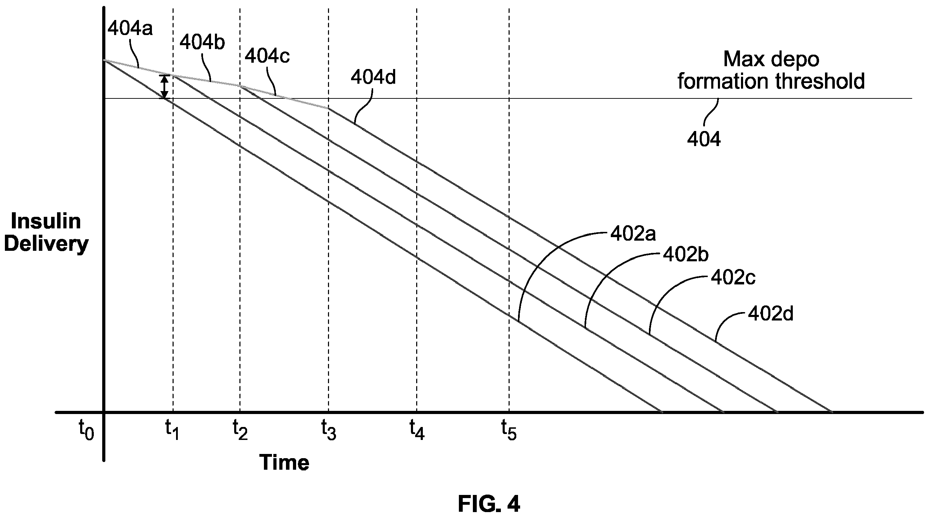

At block 302, a first aggregate IOB amount for a user is calculated based on a plurality of individual IOB estimates made over a period of time. In one example, the first aggregate IOB amount is calculated using Equation (2). FIG. 4 illustrates a plot 400 of example IOB amounts of a user over time. In one example, the first aggregate IOB amount calculated in block 302 corresponds to a first IOB curve 402a beginning at time $t_0$. As shown, the first IOB curve 402a decays at a linear rate corresponding to the linear decay curve $D_h$. It should be appreciated that in other examples, the first IOB curve 402a may decay at a non-linear rate (e.g., based on a non-linear decay curve).

In one example, the plurality of individual IOB estimates used to calculate the aggregate IOB amount were made over a period of time prior to time $t_0$. The first IOB curve 402a represents a forecasted IOB amount for the user over a plurality of control cycles. An initial control cycle begins at time $t_0$, the next control cycle begins at time $t_1$, and so on. The control cycles may be evenly distributed in time. For example, if there are 12 cycles per hour (e.g., $j$=12), a new cycle may start every 5 minutes. Of course, the number of cycles per hour may differ (e.g., every minute) or vary (e.g., every minute during one period of time and every 10 minutes during a second period of time) over time.

At block 304, the first aggregate IOB amount (e.g., the first IOB curve 402a) is compared to a maximum depot formation threshold amount. In one example, the comparison may occur at, near, or slightly before the next control cycle (e.g., time $t_1$). As shown in FIG. 4, the first IOB curve 402a may be compared to a maximum depot formation threshold 404 at (or slightly before) time $t_1$.

The calculated aggregate IOB amount being under the threshold 404 may provide an indication that the user's rate of insulin absorption is unsaturated. When unsaturated, insulin from the insulin depot may be absorbed by the user at a substantially expected rate (e.g., the linear decay curve $D_h$). Conversely, the calculated aggregate IOB amount being at or over the threshold 404 may provide an indication that the user's rate of insulin absorption is saturated. As such, the insulin from the insulin depot may be absorbed by the user at a substantially unexpected rate (e.g., at a non-linear rate and/or at a rate slower than the linear decay curve $D_h$).

In one example, the maximum formation threshold amount (or the level of the threshold 404) is set at 100 μL; however, in other examples the maximum formation threshold amount may be set at a different level, such as a reduced value of 50 μL, or an increased value of 150 μL. Accordingly, in some embodiments, the maximum formation threshold amount may be between about 20 μL to about 300 μL, about 50 μL to about 150 μL and in some embodiments between about 70 μL to about 130 μL. Other threshold amounts that are higher or lower than these examples may be used. In some examples, the maximum formation threshold amount is specific to the user. For example, factors such as the user's gender, weight, height, race, and/or other genetic or hereditary factors, the user's TDI and/or basal rate, etc., may be relevant in determining (or adjusting) the maximum formation threshold amount. In one example, a user with an increased body weight compared to those in their typical demographics may have a reduced (or increased) threshold amount. In some examples, the maximum formation threshold amount may be set or adjusted based on real-time characteristics of the user (e.g., blood pressure). For example, a user with a higher blood pressure may have increased resistance to interstitial absorption of insulin.

At block 306, in response to a determination that the first aggregate IOB amount (e.g., the first IOB curve 402a) is greater than the maximum depot formation threshold amount (e.g., the maximum depot formation threshold 404), a residual amount of insulin remaining in the first aggregate IOB amount at time $t_1$ is determined. In one example, the residual amount of insulin $I_{res}$ corresponds to the amount of insulin that has not decayed due to the saturated rate of absorption.

At block 308, a subsequent calculation of one or more individual IOB estimates is increased based on the determined residual amount of insulin $I_{res}$. In one example, the IOB estimate for the first (or oldest) control cycle is increased. For example, as shown in Equation (4) below, the residual amount of insulin $I_{res}$ from the current cycle $i$ may be added to the IOB estimate from the oldest cycle:

$$IOB'(i+1) = [I(i-1) \quad I(i-2) \ \dots \ \dots \ I(i-h \cdot j - 1) \quad I(i - h \cdot j) \qquad \text{Equation (4)}$$
$$+ I_{res}(i)] \cdot D_h$$

Alternatively, the IOB estimate for the latest (or newest) control cycle may be increased based on the determined residual amount of insulin $I_{res}$. For example, as shown in Equation (5), the residual amount of insulin $I_{res}$ from the current cycle $i$ may be added to the IOB estimate for the latest cycle:

$$IOB'(i+1) = [I(i-1) \qquad\qquad\qquad\qquad\qquad\qquad\qquad \text{Equation (5)}$$
$$+ I_{res}(i) \quad I(i-2) \ \dots \ \dots \ I(i - h \cdot j - 1) \quad I(i - h \cdot j)] \cdot D_h$$

In some examples, the entire insulin delivery history is increased based on the determined residual amount of insulin $I_{res}$. For example, as shown in Equation (6) below, the residual amount of insulin $I_{res}$ from the current cycle $i$ may be added to the complete insulin delivery history:

$$IOB'(i+1) = \left( [I(i-1)\, I(i-2)\ \ldots\ \ldots\ I(i-h\cdot j-1)\ I(i-h\cdot j)] \right. \qquad \text{Equation (6)}$$

$$\left. + \frac{I_{res}(i)}{h\cdot j} \right)\cdot D_h$$

In Equations (4), (5), and (6), $IOB'(i+1)$ represents the aggregate IOB amount for the next cycle beginning at time $t_1$, $h$ and $j$ are the same factors from earlier equations, and $I_{res}(i)$ represents the residual amount of insulin from the current cycle (e.g., from time $t_0$ to time $t_1$).

At block 310, the subsequent calculation of the one or more individual IOB estimates is used in a calculation of a second aggregate IOB amount over a second period of time. In one example, the second aggregate IOB amount over the second period of time may correspond to the aggregate IOB amount $IOB'(i+1)$ calculated using Equation (4), (5), or (6). In the example, the second IOB curve 402b beginning at time $t_1$ in FIG. 4 may correspond to the second aggregate IOB amount. As shown, the second IOB curve 402b is offset from the first IOB curve 402a by the residual insulin amount $I_{res}$.

In one example, the IOB amount at time $t_0$ (e.g., the start of the first IOB curve 402a) may be compared to the IOB amount at time $t_1$ (e.g., the start of the second IOB curve 402b) to determine the actual amount of insulin absorbed by the user. An adjusted decay curve 404a connects the first IOB curve 402a to the second IOB curve 402b. The adjusted decay curve 404a represents an estimate of the actual insulin absorption that occurred between times $t_0$ and $t_1$. The effect of the user's saturated insulin absorption rate is shown by the difference between the linear curve 402a and the adjusted curve 404a.

It should be appreciated that the method 300 can be repeated over subsequent periods of time (e.g., control cycles) to maintain an accurate representation of the user's IOB. For example, at or slightly before time $t_2$, the second aggregate IOB amount (e.g., the second IOB curve 402b) may be evaluated with respect to the maximum depot formation threshold amount (e.g., the maximum depot formation threshold 404) (block 304). In response to the second aggregate IOB amount being greater than the maximum depot formation threshold amount, a second residual amount of insulin remaining in the second aggregate IOB amount is determined (block 306). The second residual amount of insulin may be used to increase a subsequent calculation of an individual IOB estimate (block 308). The subsequent calculation of the individual IOB estimate is used to calculate a third IOB amount over a third period of time (block 310). In one example, the third IOB amount corresponds to the third IOB curve 402c in FIG. 4. An adjusted decay curve 404b connects the second IOB curve 402a to the third IOB curve 402c, representing an estimate of the actual insulin absorption that occurred between times $t_1$ and $t_2$.

Similarly, at or slightly before time $t_3$, the third aggregate IOB amount (e.g., the third IOB curve 402c) may be evaluated with respect to the maximum depot formation threshold amount (e.g., the maximum depot formation threshold 404). In response to the third aggregate IOB amount being less than the maximum depot formation threshold amount, the controller or processor may determine that the user has begun (or returned) to absorb insulin at an unsaturated absorption rate. As such, a fourth IOB amount over a fourth period of time may be calculated without incorporating a residual amount of insulin (e.g., using Equation (2)).

In a further example, the fourth IOB amount corresponds to the fourth IOB curve 402d in FIG. 4. An adjusted decay curve 404c connects the third IOB curve 402c to the fourth IOB curve 402d, representing an estimate of the actual insulin absorption that occurred between times $t_2$ and $t_3$. The fourth IOB curve 402d may be used to estimate the user's IOB amount for future cycles (e.g., time $t_5$ and so on). Once a new (or next) insulin delivery is made, the user's IOB may be revaluated relative to the maximum depot formation threshold 404.

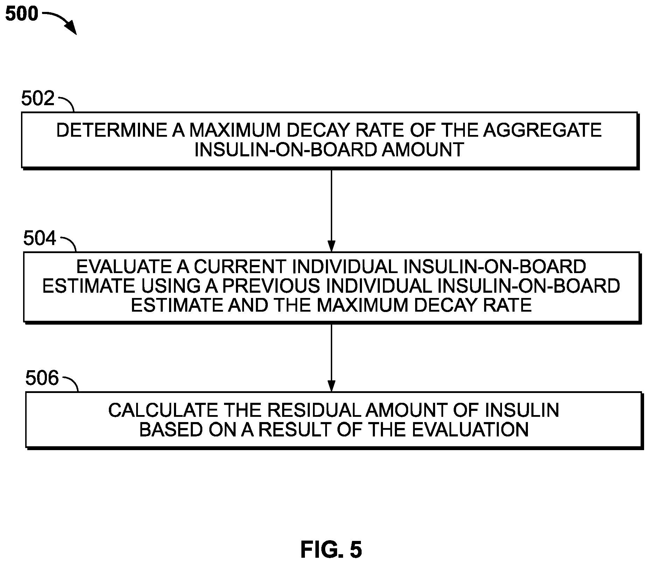

FIG. 5 illustrates a flow diagram of an example method for determining a residual amount of insulin remaining in an insulin depot (e.g., an aggregate IOB amount). In one example, the example method 500 corresponds to the step performed in block 306 of the method 300 of FIG. 3.

At block 502, a maximum decay rate of the aggregate IOB amount is determined. In one example, the maximum decay (or absorption) rate corresponds to the maximum amount of insulin that can theoretically be absorbed by the user during each control cycle. In some examples, the maximum decay rate corresponds to the rate at which insulin is absorbed by the user based on a selected time period and a preset number of units of delivered insulin. For example, the maximum decay rate may be calculated using Equation (7) below:

$$IOB_{Dmax,h} = \frac{d}{h\cdot j} \qquad \text{Equation (7)}$$

where, $IOB_{Dmax,h}$ is the maximum decay rate, $j$ is the same factor from earlier examples, and $d$ is the number of insulin units delivered over the number of hours $h$ over which insulin utilization is being forecasted. A 100 μL delivery of U-100 insulin is equivalent to 0.1 mL, or 10 units (U), of insulin. In such examples, $d$ may be set to 10. Of course, other values of $d$ may be used, i.e. $d$ may be a tunable factor.

At block 504, the IOB amount for the current cycle is evaluated using an IOB amount from a previous cycle and the maximum decay rate. For example, a time $t_1$ in FIG. 4, the IOB amount at time $t_1$ (e.g., cycle $i$) is evaluated relative to the IOB amount at time $t_0$ (e.g., cycle $i$-1) and the maximum decay rate $IOB_{Dmax,h}$. Likewise, at time $t_2$, the IOB amount at time $t_2$ is evaluated relative to the IOB amount at time $t_1$ and the maximum decay rate $IOB_{Dmax,h}$, and so on. In one example, the evaluation of the IOB amounts produces an effective IOB amount for the current cycle. The effective IOB amount for the current cycle may be produced using Equation (8) below:

$$IOB_{eff}(i) = \max\left(IOB(i), IOB(i - 1) - IOB_{Dmax,h}\right) \qquad \text{Equation (8)}$$

where $IOB_{eff}(i)$ is the effective IOB amount for the current cycle.

At block 506, the residual amount of insulin for the current cycle is calculated. In one example, the residual insulin amount $I_{res}$ is calculated by taking the difference between the IOB amount for the current cycle and the corresponding effective IOB amount. For example, the residual insulin amount $I_{res}$ may be calculated using Equation (9):

$$I_{res}(i) = IOB_{eff}(i) - IOB(i) \qquad \text{Equation (9)}$$

As described above, the residual insulin amount $I_{res}$ may be used to increase the calculation of one or more individual IOB estimates when determining the actual rate of absorption experienced by the user (e.g., block 306 of FIG. 3).

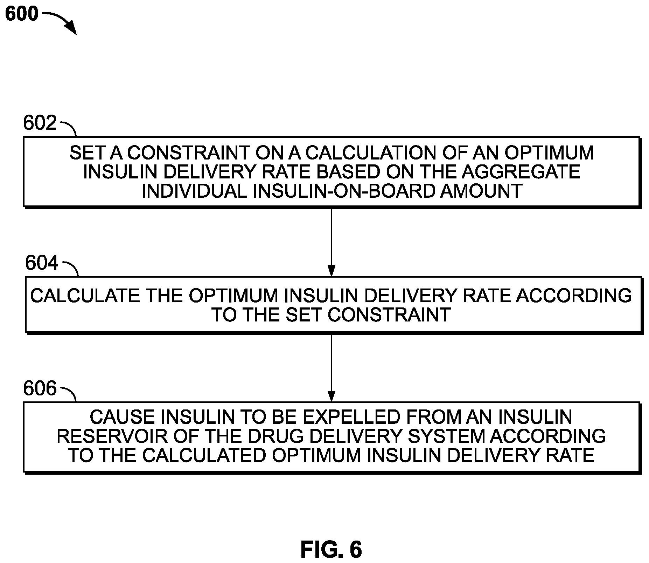

FIG. 6 illustrates a flow diagram of an example method for calculating an optimum insulin delivery rate and delivering insulin at the optimum insulin delivery rate. In one example, the method 600 is performed using the aggregate IOB amounts produced by the method 300 of FIG. 3.

For example, at block 602, a constraint is set on the optimum insulin delivery rate based on an aggregate IOB amount. In one example, the constraint corresponds to an upper IOB constraint or limit. For example, the upper IOB constraint may be calculated using Equation (10) below:

$$U_{IOB}(i) = \frac{G(i) - target(i)}{CF(i)} - IOB_{eff}(i) \qquad \text{Equation (10)}$$

where, $G(i)$ is the current glucose concentration of the user, *target*($i$) is the target glucose concentration, *CF(i)* is a correction factor, and $U_{IOB}(i)$ is the upper IOB constraint at each cycle. As described above, the user's glucose level $G(i)$ may be obtained using a continuous glucose monitor (CGM) (e.g., sensor 108 of FIG. 1). In some examples, the correction factor *CF(i)* corresponds to 1800/TDI, where TDI is the user's total daily insulin dose. The 1800/TDI may also be a tunable factor. The effective IOB amount $IOB_{eff}(i)$ may be calculated using Equation (8) (e.g., block 504 of the method 500 of FIG. 5). It should be appreciated that the constraint set on the optimum insulin delivery rate may include a lower IOB bound.

At block 604, the optimum insulin delivery rate is calculated. The optimum insulin delivery rate may be calculated on a cycle-by-cycle basis or a multi-cycle basis. In one example, an initial insulin delivery rate is determined by an automated insulin delivery (AID) device or system (e.g., systems 100, 200 of FIGS. 1-2B). In some examples, the initial insulin delivery rate is determined by the AID system to provide bolus insulin to the user. The initial insulin delivery rate may be evaluated relative to the constraint(s) determined in block 602 to calculate the optimum insulin delivery rate. For example, the constraint(s) take into account the user's maximum IOB amount and can be used to adjust or limit the insulin delivery rate such that the user does not reach or exceed the maximum IOB amount. As such, the optimum insulin delivery rate enables the AID device to provide the desired amount of insulin to the user while limiting the user's exposure to over-deliveries.

At block 606, the AID device is controlled to deliver insulin to the user according to the calculated optimum insulin delivery rate. In one example, the AID device is controlled such that insulin is expelled from an insulin reservoir of the AID device at the optimum insulin delivery rate. In some examples, the optimum insulin delivery rate corresponds to one or more control signals that control one or more components of the AID device. For example, a drive mechanism having a fluid pathway coupled to the reservoir may be operable to receive the control signal(s) and expel an amount of insulin from the reservoir based on the received control signal(s) (e.g., at the optimum insulin delivery rate).

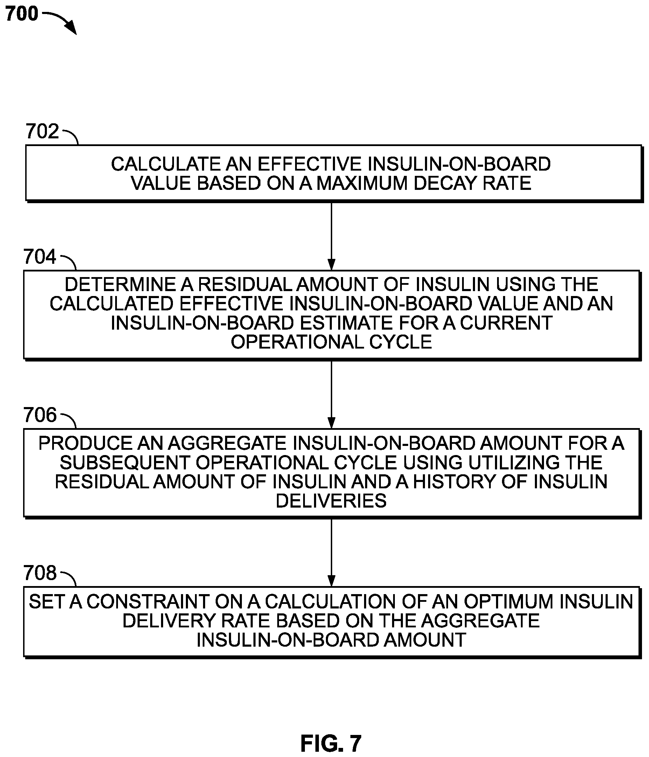

FIG. 7 illustrates a flow diagram of an example method for delivering insulin based on a constrained optimum insulin delivery rate. In one example, the method 700 is configured to be carried out, at least in part, by the system 100 of FIG. 1 or the system 200 of FIG. 2. In some examples, steps of the method 700 may be carried out by a smartphone or PDM device in communication with the AID system that includes a drug delivery device. The method 700, for example, may be performed in response to a determination that the user is experiencing a saturated rate of insulin absorption.

At block 702, an effective IOB amount is calculated based on a maximum decay rate. In one example, the maximum decay rate is calculated using Equation (7) (e.g., as done in block 502 of the method 500 of FIG. 5). Likewise, the effective IOB amount may be calculated using Equation (8) (e.g., as done in block 504 of the method 500 of FIG. 5).

At block 704, a residual amount of insulin is determined using the calculated effective IOB amount and an IOB estimate for a current operational cycle. In an example, the residual amount of insulin corresponds to the amount of insulin that has not decayed due to the saturated rate of absorption. The residual insulin amount is calculated by taking the difference between the effective IOB amount and the IOB estimate for a current operational cycle. For example, the residual amount of insulin may be calculated using Equation (9) (e.g., as done in block 506 of the method 500 of FIG. 5).

At block 706, an aggregate IOB amount for a subsequent operational cycle is produced using the residual amount of insulin and a history of insulin deliveries. In one example, the aggregate IOB amount is determined by increasing at least one individual IOB estimate from the history of insulin deliveries based on the residual amount of insulin. For example, the aggregate IOB amount may be calculated using one of Equations (4), (5), and (6) (e.g., as done in block 308 of the method 300 of FIG. 3).

At block 708, a constraint is set on a calculation of an optimum insulin delivery rate based on the aggregate IOB amount. In one example, the constraint corresponds to an upper IOB bound or limit. For example, the constraint may be calculated using Equation (10) (e.g., as done in block 602 of the method 600 of FIG. 6). The constraint may be applied to an initial delivery rate determined by the drug delivery device. By comparing the constraint to the initial delivery rate, the optimum delivery rate can be derived by preventing the user's IOB from reaching or exceeding the constraint. The optimum insulin delivery rate enables the drug delivery device to provide the desired amount of insulin to the user while limiting the user's exposure to over-deliveries.

As described above, improved techniques for determining the rate of insulin delivery from AID systems and devices are provided herein. In at least one embodiment, an aggregate IOB amount for a user is calculated based on a plurality of individual IOB estimates made over a period of time. In one example, the calculated aggregate IOB amount may be compared to a maximum depot formation threshold amount. In response to a determination that the calculated aggregate IOB amount is greater than the maximum depot formation threshold amount, a residual amount of insulin remaining in the calculated aggregate IOB amount may be determined and used to increase a subsequent calculation of an individual IOB estimate. In some examples, the subsequent calculation of the individual IOB estimate is utilized in a calculation of a further aggregate IOB amount over a subsequent period of time.

The techniques described herein for a drug delivery system (e.g., the system 100, the system 200, or any components thereof) may be implemented in hardware, software, or any combination thereof. Any component as described herein may be implemented in hardware, software, or any combination thereof. For example, the systems 100, 200 or any components thereof may be implemented in hardware, software, or any combination thereof. Software related implementations of the techniques described herein may include, but are not limited to, firmware, application specific software, or any other type of computer readable instructions that may be executed by one or more processors. Hardware related implementations of the techniques described herein may include, but are not limited to, integrated circuits (ICs), application specific ICs (ASICs), field programmable arrays (FPGAs), and/or programmable logic devices (PLDs). In some examples, the techniques described herein, and/or any system or constituent component described herein may be implemented with a processor executing computer readable instructions stored on one or more memory components.

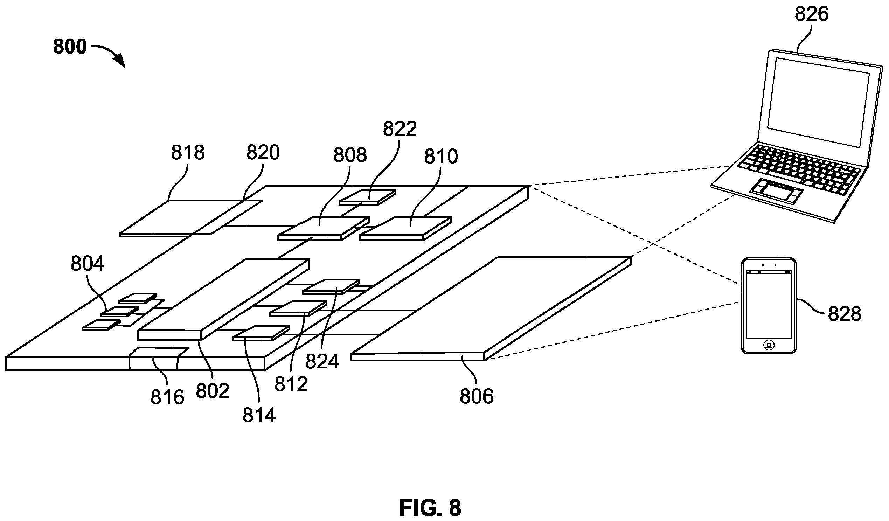

FIG. 8 shows an example of a generic computing device 800, which may be used with some of the techniques described in this disclosure (e.g., systems 100, 200 or devices in communication with the systems 100, 200). Computing device 800 may include a processor 802, a memory 804, an input/output device such as a display 806, a communication interface 808, and a transceiver 810, among other components. The device 800 may also be provided with an electronic storage device, such as a micro-drive or other device, to provide additional data and programming code storage. Each of the components 800, 802, 804, 806, 808, and 810, are interconnected using various buses, and several of the components may be mounted on a common motherboard or in other manners as appropriate.

The processor 802 can execute instructions within the computing device 800, including instructions stored in the memory 804. The processor 802 may be implemented as a chipset of chips that include separate and multiple analog and digital processors. The processor 802 may provide, for example, for coordination of the other components of the device 800, such as control of user interfaces, applications run by device 800, and wireless communication by device 800.

Processor 802 may communicate with a user through control interface 812 and display interface 814 coupled to a display 806. The display 806 may be, for example, a TFT LCD (Thin-Film-Transistor Liquid Crystal Display) or an OLED (Organic Light Emitting Diode) display, touchscreen display, or other appropriate display technology. The display interface 814 may comprise appropriate circuitry for driving the display 806 to present graphical and other information to a user. The control interface 812 may receive commands from a user and convert them for submission to the processor 802. In addition, an external interface 816 may be provided in communication with processor 802, so as to enable near area communication of device 800 with other devices. External interface 816 may provide, for example, for wired communication in some implementations, or for wireless communication in other implementations, and multiple interfaces may also be used.

The memory 804 stores information within the computing device 800. The memory 804 can be implemented as one or more of a computer-readable medium or media, a volatile memory unit or units, or a non-volatile memory unit or units. Expansion memory 818 may also be provided and connected to device 800 through expansion interface 820.

Device 800 may communicate wirelessly through communication interface 808, which may include digital signal processing circuitry where necessary. Communication interface 808 may in some cases be a cellular modem. Such communication may occur, for example, through radiofrequency transceiver 810. In addition, short-range communication may occur, such as using a Bluetooth, Wi-Fi, or other such transceiver (not shown). In addition, GPS (Global Positioning System) receiver module 822 may provide additional navigation- and location-related wireless data to device 800, which may be used as appropriate by applications running on device 800.

Device 800 may also communicate audibly using audio codec 824, which may receive spoken information from a user and convert it to usable digital information. Audio codec 824 may likewise generate audible sound for a user, such as through a speaker, e.g., in a handset of device 800.

The computing device 800 may be implemented in a number of different forms, as shown in FIG. 8. For example, it may be implemented as a computer (e.g., laptop) 826. It may also be implemented as part of a smartphone (or PDM device) 828, smart watch, tablet, personal digital assistant, or another similar mobile device.

While this specification contains many specific implementation details, these should not be construed as limitations on the scope of any inventions or of what may be claimed, but rather as descriptions of features specific to particular implementations of particular inventions. Certain features that are described in this specification in the context of separate implementations can also be implemented in combination in a single implementation. Conversely, various features that are described in the context of a single implementation can also be implemented in multiple implementations separately or in any suitable subcombination. Moreover, although features may be described above as acting in certain combinations and even initially claimed as such, one or more features from a claimed combination can in some cases be excised from the combination, and the claimed combination may be directed to a subcombination or variation of a subcombination.

Similarly, while operations are depicted in the drawings in a particular order, this should not be understood as requiring that such operations be performed in the particular order shown or in sequential order, or that all illustrated operations be performed, to achieve desirable results. In certain circumstances, multitasking and parallel processing may be advantageous. Moreover, the separation of various system components in the implementations described above should not be understood as requiring such separation in all implementations, and it should be understood that the described program components and systems can generally be integrated together in a single software product or packaged into multiple software products.

Thus, particular implementations of the subject matter have been described. Other implementations are within the scope of the following claims. In some cases, the actions recited in the claims can be performed in a different order and still achieve desirable results. In addition, the processes depicted in the accompanying figures do not necessarily require the particular order shown, or sequential order, to achieve desirable results.

Certain examples of the present disclosed subject matter were described above. It is, however, expressly noted that the present disclosed subject matter is not limited to those examples, but rather the intention is that additions and modifications to what was expressly described herein are also included within the scope of the disclosed subject matter. Moreover, it is to be understood that the features of the various examples described herein were not mutually exclusive and may exist in various combinations and permutations, even if such combinations or permutations were not made express herein, without departing from the spirit and scope of the disclosed subject matter. In fact, variations, modifications, and other implementations of what was described herein will occur to those of ordinary skill in the art without departing from the spirit and the scope of the disclosed subject matter. As such, the disclosed subject matter is not to be defined only by the preceding illustrative description.

Program aspects of the technology may be thought of as "products" or "articles of manufacture" typically in the form of executable code and/or associated data that is carried on or embodied in a type of machine readable medium. Storage type media include any or all of the tangible memory of the computers, processors or the like, or associated modules thereof, such as various semiconductor memories, tape drives, disk drives and the like, which may provide non-transitory storage at any time for the software programming. It is emphasized that the Abstract of the Disclosure is provided to allow a reader to quickly ascertain the nature of the technical disclosure. It is submitted with the understanding that it will not be used to interpret or limit the scope or meaning of the claims. In addition, in the foregoing Detailed Description, various features are grouped together in a single example for streamlining the disclosure. This method of disclosure is not to be interpreted as reflecting an intention that the claimed examples require more features than are expressly recited in each claim. Rather, as the following claims reflect, inventive subject matter lies in less than all features of a single disclosed example. Thus, the following claims are hereby incorporated into the Detailed Description, with each claim standing on its own as a separate example. In the appended claims, the terms "including" and "in which" are used as the plain-English equivalents of the respective terms "comprising" and "wherein," respectively. Moreover, the terms "first," "second," "third," and so forth, are used merely as labels and are not intended to impose numerical requirements on their objects.

The foregoing description of example examples has been presented for the purposes of illustration and description. It is not intended to be exhaustive or to limit the present disclosure to the precise forms disclosed. Many modifications and variations are possible in light of this disclosure. It is intended that the scope of the present disclosure be limited not by this detailed description, but rather by the claims appended hereto. Future filed applications claiming priority to this application may claim the disclosed subject matter in a different manner and may generally include any set of one or more limitations as variously disclosed or otherwise demonstrated herein.

Although the present invention is defined in the attached claims, it should be understood that the present invention can also (alternatively) be defined in accordance with the following embodiments:

1. A drug delivery system, comprising:

a memory storing programming code operable to enable delivery of insulin;
a processor operable to execute the programming code, wherein the programming code causes the processor to:

calculate a first aggregate insulin-on-board amount for a user based on a plurality of individual insulin-on-board estimates made over a first period of time;
determine that the first aggregate insulin-on-board amount is greater than a maximum depot formation threshold amount;
determine a first residual amount of insulin remaining in the first aggregate insulin-on-board amount;
increase a subsequent calculation of an individual insulin-on-board estimate based on the first residual amount of insulin; and
utilize the subsequent calculation of the individual insulin-on-board estimate in a calculation of a second aggregate insulin-on-board amount over a second period of time.

2. The drug delivery system of embodiment 1, wherein the programming code further causes the processor to:

evaluate the second aggregate insulin-on-board amount with respect to the maximum depot formation threshold amount; and
in response to the second aggregate insulin-on-board amount being greater than the maximum depot formation threshold amount, determine a second residual amount of insulin remaining in the second the aggregate insulin-on-board amount.

3. The drug delivery system of embodiment 2, wherein the programming code further causes the processor to:

increase a subsequent calculation of another individual insulin-on-board estimate using the second residual amount; and
utilize the subsequent calculation of the other individual insulin-on-board estimate in a calculation of a third insulin-on-board amount over a third period of time.

4. The drug delivery system of embodiment 1, wherein the programming code further causes the processor, when determining the first residual amount of insulin, to:

determine a maximum decay rate of the first aggregate insulin-on-board amount;
evaluate a current individual insulin-on-board estimate using a previous individual insulin-on-board estimate and the maximum decay rate; and
calculate the first residual amount of insulin based on a result of the evaluation.

5. The drug delivery system of embodiment 4, wherein the programming code further causes the processor, when determining the first residual amount of insulin, to:
calculate the maximum decay rate of the aggregate insulin-on-board amount, wherein the maximum decay rate is a rate at which the insulin is absorbed by a user based on a selected time period and a preset number of units of the delivered insulin.

6. The drug delivery system of embodiment 1, wherein the programming code further causes the processor to:

   set a constraint on a calculation of an optimum insulin delivery rate based on the subsequent calculation of the individual insulin-on-board estimate;
   calculate the optimum insulin delivery rate according to the set constraint; and
   cause insulin to be expelled from an insulin reservoir of the drug delivery system according to the calculated optimum insulin delivery rate.

7. The drug delivery system of embodiment 1, wherein the programming code further causes the processor to:

   set a constraint on a calculation of an optimum insulin delivery rate based on the calculation of the second aggregate insulin-on-board amount;
   calculate the optimum insulin delivery rate according to the set constraint; and
   cause insulin to be expelled from an insulin reservoir of the drug delivery system according to the calculated optimum insulin delivery rate.

8. The drug delivery system of embodiment 1, further comprising:
a wearable drug delivery device including:

   a reservoir operable to contain the insulin, and
   a drive mechanism having a fluid pathway coupled to the reservoir operable to receive a control signal and expel an amount of insulin from the reservoir based on the received control signal.

9. The drug delivery system of embodiment 8, wherein the wearable drug delivery device further includes:

   the memory and the processor, and
   the processor is coupled to the drive mechanism and further operable to output the control signal to the drive mechanism.

10. A non-transitory computer readable medium embodied with programming code that causes a processor to:

   calculate a first aggregate insulin-on-board amount for a user based on a plurality of individual insulin-on-board estimates made over a first period of time;
   determine that the first aggregate insulin-on-board amount is greater than a maximum depot formation threshold amount;
   determine a first residual amount of insulin remaining in the first aggregate insulin-on-board amount;
   increase a subsequent calculation of an individual insulin-on-board estimate based on the first residual amount of insulin; and
   utilize the subsequent calculation of the individual insulin-on-board estimate in a calculation of a second aggregate insulin-on-board amount over a second period of time.

11. The non-transitory computer readable medium of embodiment 10, wherein the programming code further causes the processor to:

   evaluate the calculation of the second aggregate insulin-on-board amount with respect to the maximum depot formation threshold amount; and
   in response to the calculation of the second aggregate insulin-on-board amount being greater than the maximum depot formation threshold amount, determine a second residual amount of insulin remaining in the calculation of the second aggregate insulin-on-board amount.

12. The non-transitory computer readable medium of embodiment 11, wherein the programming code further causes the processor to:

   increase an ensuing calculation of another individual insulin-on-board estimate using the determined second residual amount; and
   utilize the ensuing calculation of the other individual insulin-on-board estimate in a calculation of a third aggregate insulin-on-board amount over a third period of time.

13. The non-transitory computer readable medium of embodiment 12, wherein the programming code further causes the processor, when determining the residual amount of insulin, to:

determine a maximum decay rate of the first aggregate individual insulin-on-board amount;
evaluate a current individual insulin-on-board estimate using a previous individual insulin-on-board estimate and the maximum decay rate; and
calculate the first residual amount of insulin based on a result of the evaluation.

14. The non-transitory computer readable medium of embodiment 13, wherein the programming code further causes the processor, when determining the first residual amount of insulin, to:
calculate the maximum decay rate of the first aggregate insulin-on-board amount, wherein the maximum decay rate is a rate at which the insulin is absorbed by a user based on a selected time period and a preset number of units of the delivered insulin.

15. The non-transitory computer readable medium of embodiment 10, wherein the programming code further causes the processor to:

set a constraint on a calculation of an optimum insulin delivery rate based on the subsequent calculation of the individual insulin-on-board estimate;
calculate the optimum insulin delivery rate according to the set constraint; and
cause insulin to be expelled from a reservoir of a wearable drug delivery device according to the calculated optimum insulin delivery rate.

16. The non-transitory computer readable medium of embodiment 10, wherein the programming code further causes the processor to:

set a constraint on a calculation of an optimum insulin delivery rate based on the calculation of the second aggregate individual insulin-on-board amount;
calculate the optimum insulin delivery rate according to the set constraint; and
cause insulin to be expelled from a reservoir of a wearable drug delivery device according to the calculated optimum insulin delivery rate.

17. A drug delivery system, comprising:

a memory storing programming code operable to enable delivery of insulin;
a processor operable to execute the programming code, wherein the programming code causes the processor to:

calculate an effective insulin-on-board value based on a maximum decay rate;
determine a residual amount of insulin using the calculated effective insulin-on-board value and an insulin-on-board estimate for a current operational cycle;
produce an aggregate insulin-on-board amount for a subsequent operational cycle using utilizing the residual amount of insulin and a history of insulin deliveries; and
set a constraint on a calculation of an optimum insulin delivery rate based on the aggregate insulin-on-board amount.

18. The drug delivery system of embodiment 17, wherein the programming code further causes the processor to:
calculate the maximum decay rate, wherein the maximum decay rate is a rate at which the insulin is absorbed by a user based on a selected time period and a preset number of units of the delivered insulin.

19. The drug delivery system of embodiment 17, wherein the programming code further causes the processor to:
calculate the optimum insulin delivery rate according to the set constraint.

20. The drug delivery system of embodiment 17, wherein the programming code further causes the processor, when producing an aggregate insulin-on-board amount, to:
calculate the aggregate insulin-on-board amount based on a plurality of individual insulin-on-board estimates made over a period of time.

**Claims**

1. A drug delivery system, comprising:

   a memory storing programming code operable to enable delivery of insulin;
   a processor operable to execute the programming code, wherein the programming code causes the processor to:

   calculate a first aggregate insulin-on-board amount for a user based on a plurality of individual insulin-on-board estimates made over a first period of time;
   determine that the first aggregate insulin-on-board amount is greater than a maximum depot formation threshold amount;
   determine a first residual amount of insulin remaining in the first aggregate insulin-on-board amount;
   increase a subsequent calculation of an individual insulin-on-board estimate based on the first residual amount of insulin; and
   utilize the subsequent calculation of the individual insulin-on-board estimate in a calculation of a second aggregate insulin-on-board amount over a second period of time.

2. The drug delivery system of claim 1, wherein the programming code further causes the processor to:

   evaluate the second aggregate insulin-on-board amount with respect to the maximum depot formation threshold amount; and
   in response to the second aggregate insulin-on-board amount being greater than the maximum depot formation threshold amount, determine a second residual amount of insulin remaining in the second the aggregate insulin-on-board amount.

3. The drug delivery system of claim 2, wherein the programming code further causes the processor to:

   increase a subsequent calculation of another individual insulin-on-board estimate using the second residual amount; and
   utilize the subsequent calculation of the other individual insulin-on-board estimate in a calculation of a third insulin-on-board amount over a third period of time.

4. The drug delivery system of any one of claims 1 to 3, wherein the programming code further causes the processor, when determining the first residual amount of insulin, to:

   determine a maximum decay rate of the first aggregate insulin-on-board amount;
   evaluate a current individual insulin-on-board estimate using a previous individual insulin-on-board estimate and the maximum decay rate; and
   calculate the first residual amount of insulin based on a result of the evaluation.

5. The drug delivery system of claim 4, wherein the programming code further causes the processor, when determining the first residual amount of insulin, to:
   calculate the maximum decay rate of the aggregate insulin-on-board amount, wherein the maximum decay rate is a rate at which the insulin is absorbed by a user based on a selected time period and a preset number of units of the delivered insulin.

6. The drug delivery system of any one of claims 1 to 5, wherein the programming code further causes the processor to:

   set a constraint on a calculation of an optimum insulin delivery rate based on the subsequent calculation of the individual insulin-on-board estimate;
   calculate the optimum insulin delivery rate according to the set constraint; and
   cause insulin to be expelled from an insulin reservoir of the drug delivery system according to the calculated optimum insulin delivery rate.

7. The drug delivery system of any one of claims 1 to 6, wherein the programming code further causes the processor to:

   set a constraint on a calculation of an optimum insulin delivery rate based on the calculation of the second aggregate insulin-on-board amount;

calculate the optimum insulin delivery rate according to the set constraint; and
cause insulin to be expelled from an insulin reservoir of the drug delivery system according to the calculated optimum insulin delivery rate.

8. The drug delivery system of any one of claims 1 to 7, further comprising:
a wearable drug delivery device including:

a reservoir operable to contain the insulin, and
a drive mechanism having a fluid pathway coupled to the reservoir operable to receive a control signal and expel an amount of insulin from the reservoir based on the received control signal.

9. The drug delivery system of claim 8, wherein the wearable drug delivery device further includes:

the memory and the processor, and
the processor is coupled to the drive mechanism and further operable to output the control signal to the drive mechanism.

10. A non-transitory computer readable medium embodied with programming code that causes a processor to:

calculate a first aggregate insulin-on-board amount for a user based on a plurality of individual insulin-on-board estimates made over a first period of time;
determine that the first aggregate insulin-on-board amount is greater than a maximum depot formation threshold amount;
determine a first residual amount of insulin remaining in the first aggregate insulin-on-board amount;
increase a subsequent calculation of an individual insulin-on-board estimate based on the first residual amount of insulin; and
utilize the subsequent calculation of the individual insulin-on-board estimate in a calculation of a second aggregate insulin-on-board amount over a second period of time.

11. The non-transitory computer readable medium of claim 10, wherein the programming code further causes the processor to:

evaluate the calculation of the second aggregate insulin-on-board amount with respect to the maximum depot formation threshold amount; and
in response to the calculation of the second aggregate insulin-on-board amount being greater than the maximum depot formation threshold amount, determine a second residual amount of insulin remaining in the calculation of the second aggregate insulin-on-board amount.

12. The non-transitory computer readable medium of claim 11, wherein the programming code further causes the processor to:

increase an ensuing calculation of another individual insulin-on-board estimate using the determined second residual amount; and
utilize the ensuing calculation of the other individual insulin-on-board estimate in a calculation of a third aggregate insulin-on-board amount over a third period of time.

13. The non-transitory computer readable medium of claim 12, wherein the programming code further causes the processor, when determining the residual amount of insulin, to:

determine a maximum decay rate of the first aggregate individual insulin-on-board amount;
evaluate a current individual insulin-on-board estimate using a previous individual insulin-on-board estimate and the maximum decay rate; and
calculate the first residual amount of insulin based on a result of the evaluation.

14. The non-transitory computer readable medium of claim 13, wherein the programming code further causes the processor, when determining the first residual amount of insulin, to:
calculate the maximum decay rate of the first aggregate insulin-on-board amount, wherein the maximum decay rate is a rate at which the insulin is absorbed by a user based on a selected time period and a preset number of units of the delivered insulin.

15. The non-transitory computer readable medium of any one of claims 10 to 14, wherein the programming code further causes the processor to:

set a constraint on a calculation of an optimum insulin delivery rate based on the subsequent calculation of the individual insulin-on-board estimate;
calculate the optimum insulin delivery rate according to the set constraint; and
cause insulin to be expelled from a reservoir of a wearable drug delivery device according to the calculated optimum insulin delivery rate.

16. The non-transitory computer readable medium of any one of claims 10 to 15, wherein the programming code further causes the processor to:

set a constraint on a calculation of an optimum insulin delivery rate based on the calculation of the second aggregate individual insulin-on-board amount;
calculate the optimum insulin delivery rate according to the set constraint; and
cause insulin to be expelled from a reservoir of a wearable drug delivery device according to the calculated optimum insulin delivery rate.

17. A drug delivery system, comprising:

a memory storing programming code operable to enable delivery of insulin;
a processor operable to execute the programming code, wherein the programming code causes the processor to:

calculate an effective insulin-on-board value based on a maximum decay rate;
determine a residual amount of insulin using the calculated effective insulin-on-board value and an insulin-on-board estimate for a current operational cycle;
produce an aggregate insulin-on-board amount for a subsequent operational cycle using utilizing the residual amount of insulin and a history of insulin deliveries; and
set a constraint on a calculation of an optimum insulin delivery rate based on the aggregate insulin-on-board amount.

18. The drug delivery system of claim 17, wherein the programming code further causes the processor to:
calculate the maximum decay rate, wherein the maximum decay rate is a rate at which the insulin is absorbed by a user based on a selected time period and a preset number of units of the delivered insulin.

19. The drug delivery system of claim 17 or 18, wherein the programming code further causes the processor to:
calculate the optimum insulin delivery rate according to the set constraint.

20. The drug delivery system of any one of claims 17 to 19, wherein the programming code further causes the processor, when producing an aggregate insulin-on-board amount, to:
calculate the aggregate insulin-on-board amount based on a plurality of individual insulin-on-board estimates made over a period of time.

**FIG. 1**

EP 4 369 350 A1

**FIG. 2A**

EP 4 369 350 A1

FIG. 2B

300

302

CALCULATE A FIRST AGGREGATE INSULIN-ON-BOARD AMOUNT FOR A USER BASED ON A PLURALITY OF INDIVIDUAL INSULIN-ON-BOARD ESTIMATES MADE OVER A FIRST PERIOD OF TIME

304

DETERMINE THAT THE FIRST AGGREGATE INSULIN-ON-BOARD AMOUNT IS GREATER THAN A MAXIMUM DEPOT FORMATION THRESHOLD AMOUNT

306

DETERMINE A FIRST RESIDUAL AMOUNT OF INSULIN REMAINING IN THE FIRST AGGREGATE INSULIN-ON-BOARD AMOUNT

308

INCREASE A SUBSEQUENT CALCULATION OF AN INDIVIDUAL INSULIN-ON-BOARD ESTIMATE BASED ON THE FIRST RESIDUAL AMOUNT OF INSULIN

310

UTILIZE THE SUBSEQUENT CALCULATION OF THE INDIVIDUAL INSULIN-ON-BOARD ESTIMATE IN A CALCULATION OF A SECOND AGGREGATE INSULIN-ON-BOARD AMOUNT OVER A SECOND PERIOD OF TIME

FIG. 3

**FIG. 4**

500

502

DETERMINE A MAXIMUM DECAY RATE OF THE AGGREGATE
INSULIN-ON-BOARD AMOUNT

504

EVALUATE A CURRENT INDIVIDUAL INSULIN-ON-BOARD
ESTIMATE USING A PREVIOUS INDIVIDUAL INSULIN-ON-BOARD
ESTIMATE AND THE MAXIMUM DECAY RATE

506

CALCULATE THE RESIDUAL AMOUNT OF INSULIN
BASED ON A RESULT OF THE EVALUATION

FIG. 5

600 ⟍

602 ⟍
SET A CONSTRAINT ON A CALCULATION OF AN OPTIMUM INSULIN DELIVERY RATE BASED ON THE AGGREGATE INDIVIDUAL INSULIN-ON-BOARD AMOUNT

604 ⟍
CALCULATE THE OPTIMUM INSULIN DELIVERY RATE ACCORDING TO THE SET CONSTRAINT

606 ⟍
CAUSE INSULIN TO BE EXPELLED FROM AN INSULIN RESERVOIR OF THE DRUG DELIVERY SYSTEM ACCORDING TO THE CALCULATED OPTIMUM INSULIN DELIVERY RATE

**FIG. 6**

**700 ⟍**

**702 ⟍**
CALCULATE AN EFFECTIVE INSULIN-ON-BOARD
VALUE BASED ON A MAXIMUM DECAY RATE

**704 ⟍**
DETERMINE A RESIDUAL AMOUNT OF INSULIN USING THE
CALCULATED EFFECTIVE INSULIN-ON-BOARD VALUE AND AN
INSULIN-ON-BOARD ESTIMATE FOR A CURRENT
OPERATIONAL CYCLE

**706 ⟍**
PRODUCE AN AGGREGATE INSULIN-ON-BOARD AMOUNT FOR A
SUBSEQUENT OPERATIONAL CYCLE USING UTILIZING THE
RESIDUAL AMOUNT OF INSULIN AND A HISTORY OF INSULIN
DELIVERIES

**708 ⟍**
SET A CONSTRAINT ON A CALCULATION OF AN OPTIMUM INSULIN
DELIVERY RATE BASED ON THE AGGREGATE
INSULIN-ON-BOARD AMOUNT

**FIG. 7**

FIG. 8

Europäisches
Patentamt

European
Patent Office

Office européen
des brevets

## EUROPEAN SEARCH REPORT

**Application Number**

**EP 23 20 9641**

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br>A | US 2014/066886 A1 (ROY ANIRBAN [US] ET AL)<br>6 March 2014 (2014-03-06)<br>* paragraphs [0608], [0611] – [0612], [0614] *<br>----- | 17-20<br><br>1-16 | INV.<br>G16H20/17<br>G16H40/63 |

TECHNICAL FIELDS
SEARCHED (IPC)

G16H

The present search report has been drawn up for all claims

| Place of search | Date of completion of the search | Examiner |
|---|---|---|
| Munich | 28 March 2024 | Samulowitz, Michael |

EPO FORM 1503 03.82 (P04C01)

## ANNEX TO THE EUROPEAN SEARCH REPORT
## ON EUROPEAN PATENT APPLICATION NO.

EP 23 20 9641

This annex lists the patent family members relating to the patent documents cited in the above-mentioned European search report.
The members are as contained in the European Patent Office EDP file on
The European Patent Office is in no way liable for these particulars which are merely given for the purpose of information.

28-03-2024

| Patent document cited in search report | Publication date | Patent family member(s) | Publication date |
|---|---|---|---|
| US 2014066886 A1 | 06-03-2014 | NONE | |

For more details about this annex : see Official Journal of the European Patent Office, No. 12/82

EPO FORM P0459

**EP 4 369 350 A1**

**Patent documents cited in the description**

- US 63383595